# EUROPÄISCHE PATENTSCHRIFT

(11) **EP 1 453 778 B1**
(45) Veröffentlichungstag und Bekanntmachung des Hinweises auf die Patenterteilung: **29.03.2006**
(21) Anmeldenummer: 02804222.4
(22) Anmeldetag: 04.12.2002
(51) Int. Cl.: C07C 19/045, C07C 21/06

(54) **VERFAHREN ZUR HERSTELLUNG VON 1,2-DICHLORETHAN**
METHOD FOR THE PRODUCTION OF 1,2-DICHLOROETHANE
PROCEDE POUR PRODUIRE DU 1,2-DICHLOROETHANE

(30) Priorität: 05.12.2001 DE 10159615
(43) Veröffentlichungstag der Anmeldung: 08.09.2004
(73) Patentinhaber: BASF Aktiengesellschaft, 67056 Ludwigshafen (DE)
(72) Erfinder: HARTH, Klaus, 67317 Altleiningen (DE); SCHINDLER, Götz-Peter, 68219 Mannheim (DE); WALSDORFF, Christian, 67059 Ludwigshafen (DE); KUHRS, Christian, 69115 Heidelberg (DE); SIMON, Falk, 64625 Bensheim (DE)
(74) Vertreter: Hörschler, Wolfram Johannes
(86) Internationale Anmeldenummer: PCT/EP2002/013729
(87) Internationale Veröffentlichungsnummer: WO 2003/048088

(56) Entgegenhaltungen:
- WO-A-00/26164
- DE-A- 1 443 707

## Beschreibung

Die Erfindung betrifft ein Verfahren zur Herstellung von 1,2-Dichlorethan und von Vinylchlorid.

1,2-Dichlorethan wird durch Oxichlorierung von Ethen mit HCl und Sauerstoff oder durch Direktchlorierung von Ethen mit Chlor hergestellt. 1,2-Dichlorethan wird ganz überwiegend durch Pyrolyse zu Vinylchlorid weiterverarbeitet, wobei HCl frei wird. Üblicherweise werden Oxichlorierung und Direktchlorierung parallel durchgeführt und wird der in der Dichlorethan-Pyrolyse gewonnene Chlorwasserstoff in der Oxichlorierung eingesetzt.

Ethen wird überwiegend durch thermische Spaltung (Cracken) gesättigter Kohlenwasserstoffe hergestellt. Dabei fällt ein Kohlenwasserstoff-Gemisch aus Methan, Ethan, Ethen, Acetylen, Propan, Propen, Butenen, Butadien, C₅- und höheren Kohlenwasserstoffen an. Die Crackgase müssen einer vielstufigen Aufarbeitung unterworfen werden, ehe die reinen Endprodukte vorliegen. Die Trennung von Ethan und Ethen erfordert Kolonnen mit 80 bis 100 Böden.

Da Ethen und Propen beim Cracken von Naphta in der Regel gemeinsam anfallen, ist die Produktionsmenge des einen Produktes stets an die Produktionsmenge des anderen Produktes gekoppelt.

Ethen kann ferner durch Dehydrierung von Ethan oder aus Raffineriegasen gewonnen werden. Die Gewinnung von Ethen erfordert hierbei die aufwendige und energieintensive Abtrennung des Ethens von nicht umgesetztem Ethan und Nebenprodukten.

1,2-Dichlorethan kann durch Chlorierung von Ethen hergestellt werden. Die Chlorierung kann entweder als direkte Chlorierung mittels Chlor oder als Oxichlorierung mittels Chlorwasserstoff und Sauerstoff durchgeführt werden. 1,2-Dichlorethan wird ganz überwiegend unter Abspaltung von Chlorwasserstoff zu Vinylchlorid weiterverarbeitet. Die Oxichlorierung von Ethen hat in der chemischen Industrie darüber hinaus eine wichtige Bedeutung insofern, als sie eine kostengünstige Weiterverwertung von Chlorwasserstoff aus Verfahren, in denen Chlorwasserstoff als Koppelprodukt anfällt, beispielsweise phosgenbasierte Verfahren zur Herstellung von Isocyanaten, ermöglicht.

Nachteilig an den aus dem Stand der Technik bekannten Verfahren zur Herstellung von 1,2-Dichlorethan aus Ethen sind die hohen Kosten des Einsatzstoffes Ethen.

Ferner sind Verfahren bekannt, in denen Vinylchlorid durch Oxichlorierung von Ethan gewonnen wird. Ein solches Verfahren wird beispielsweise in der WO 95/07249 beschrieben. Das dort beschriebene sogenannte EVC-Verfahren hat jedoch den Nachteil, dass es drei verschiedene Reaktorstufen benötigt und somit aufwendig ist. Außerdem wird als Quelle für Chlor relativ teures elementares Chlor (Cl₂) eingesetzt, so dass das Verfahren keine Möglichkeit der Weiterverwertung für in anderen Verfahren anfallenden Chlorwasserstoff bietet. Schließlich muss bei diesem Verfahren bei verglichen mit der Oxichlorierung von Ethen wesentlich höheren Temperaturen gearbeitet werden, was wegen der verwendeten kupferhaltigen Katalysatoren das Risiko der Bildung von Dioxinen erheblich erhöht.

DE-A-1443707 beschreibt ein Verfahren zur Herstellung von 1,2-Dichlorethan durch oxydative Dehydrierung von Ethan und Oxychlorierung des gebildeten ethylenhaltigen Dehydrierungsprodukts. WO 00/26164 beschreibt ein Verfahren zur Herstellung von 1,2-Dichlorethan durch Cracken von Ethan und Chlorierung des gebildeten Ethylens.

Allen Verfahren der Oxichlorierung von Ethan zu Vinylchlorid ist gemeinsam, dass sie sich nicht in bestehenden Anlagen zur Herstellung von Vinylchlorid aus Ethen durchführen lassen und deshalb umfangreiche Neuinvestitionen erforderlich machen.

Aufgabe der Erfindung ist, die Herstellung von 1,2-Dichlorethan und des Folgeproduktes Vinylchlorid auf eine neue Rohstoffbasis zu stellen. Insbesondere ist es Aufgabe der Erfindung, ein Verfahren zur Herstellung von 1,2-Dichlorethan bzw. Vinylchlorid bereitzustellen, das von Ethan als Rohstoff ausgeht und das sich durch Erweiterung von existierenden Verfahren zur Herstellung von 1,2-Dichlorethan bzw. Vinylchlorid, die von Ethylen als Rohstoff ausgehen, realisieren lässt.

Gelöst wird die Aufgabe durch ein Verfahren zur Herstellung von 1,2-Dichlorethan und Vinylchlorid mit den Schritten
(A) Einspeisung eines Ethan enthaltenden Einsatzgasstroms in eine Dehydrierzone, Dehydrierung von Ethan zu Ethen unter Erhalt eines Produktgasstromes enthaltend Ethan, Ethen und Nebenbestandteile,
(B1) Einspeisung eines ersten Teilstroms des Ethan und Ethen enthaltenden Dehydrier-Produktgasstroms, gegebenenfalls nach Abtrennung von Nebenbestandteilen, in eine Oxichlorierungszone, Oxichlorierung von Ethen zu 1,2-Dichlorethan unter Erhalt eines 1,2-Dichlorethan, Ethan und gegebenenfalls weitere Nebenbestandteile enthaltenden Produktgasstroms, Gewinnung von 1,2-Dichlorethan und eines Ethan enthaltenden Kreisgasstroms und Rückführung des Ethan enthaltenden Kreisgasstroms in die Ethan-Dehydrierung,
(B2) Einspeisung eines zweiten Teilstroms des Ethan und Ethen enthaltenden Dehydrier-Produktgasstroms, gegebenenfalls nach Abtrennung von Nebenbestandteilen, in eine Direktchlorierungszone, direkte Chlorierung von Ethen zu 1,2-Dichlorethan unter Erhalt eines 1,2-Dichlorethan, Ethan und gegebenenfalls weitere Nebenbestandteile enthaltenden Produktgasstrom, Gewinnung von 1,2-Dichlorethan und eines Ethan enthaltenden Kreisgasstroms und Rückführung des Ethan enthaltenden Kreisgasstroms in die Ethan-Dehydrierung.
(C) 1,2-Dichlorethan aus der Oxichlorierung und der Direktchlorierung in eine Pyrolysezone eingespeist wird, 1,2-Dichlorethan zu Vinylchlorid pyrolysiert wird, wobei ein Vinylchlorid, Chlorwasserstoff, 1,2-Dichlorethan und gegebenenfalls Nebenbestandteile enthaltenden Produktgasstrom erhalten wird, Chlorwasserstoff und Vinylchlorid abgetrennt und Chlorwasserstoff in die Oxichlorierung zurückgeführt wird

Das erfindungsgemäße Verfahren lässt sich ohne weiteres durch Umrüstung bestehender Anlagen zur Herstellung von 1,2-Dichlorethan aus Ethen realisieren. Es zeichnet sich dadurch aus, dass Ethen in einer der Ethen-Chlorierung vorgelagerten Stufe durch Dehydrierung von Ethan gewonnen wird, die Ethen-Chlorierung in Gegenwart von nicht umgesetztem Ethan sowie gegebenenfalls weiterer Nebenbestandteile aus der Ethan-Dehydrierung durchgeführt und das nicht umgesetzte Ethan als Kreisgas in die Ethan-Dehydrierung zurückgeführt wird.

In einem Verfahrensteil (A) wird Ethan zu Ethen dehydriert.

Die Dehydrierung von Ethan zu Ethen kann durch thermische Spaltung (Cracken) von Ethan durchgeführt werden. Dabei wird beispielsweise ein Produktgasgemisch enthaltend 2,7 Gew.-% Wasserstoff, 9,5 Gew.-% Methan, 58,5 Gew.-% Ethen, 23,2 Gew.-% Ethan, 2,3 Gew.-% Propen und 3,8 Gew.- C₄⁺-Kohlenwassersoffe erhalten.

Ethan kann durch oxidative Dehydrierung zu Ethen dehydriert werden.

Die oxidative Ethan-Dehydrierung kann beispielsweise, wie in US 4,250,346 beschrieben, an Mo/V-Mischoxid-Katalysatoren oder, wie in WO 00/48971 beschrieben, an NiO enthaltenden Katalysatoren bei Temperaturen von 300 bis 500 °C und Ethan-Umsätzen von 10 bis 20% durchgeführt werden.

Bevorzugt wird die Alkan-Dehydrierung als nicht-oxidative katalytische Dehydrierung durchgeführt. Dabei wird Ethan in einem Dehydrierreaktor an einem dehydrieraktiven Katalysator teilweise zu Ethen dehydriert. Bei der Dehydrierung fällt neben Wasserstoff in geringen Mengen Methan als Crackprodukt des Ethans an. Je nach Fahrweise der Dehydrierung können außerdem Kohlenstoffoxide (CO, CO₂), Wasser und Stickstoff im Produktgasgemisch der Ethan-Dehydrierung enthalten sein. Daneben liegt im Produktgasgemisch nicht umgesetztes Ethan vor.

Die katalytische Ethan-Dehydrierung kann mit oder ohne sauerstoffhaltigem Gas als Co-Feed durchgeführt werden.

Die katalytische Ethan-Dehydrierung kann grundsätzlich in allen aus dem Stand der Technik bekannten Reaktortypen und Fahrweisen durchgeführt werden. Eine vergleichsweise ausführliche Beschreibung von erfindungsgemäß geeigneten Dehydrierverfahren enthält auch "Catalytica® Studies Division, Oxidative Dehydrogenation and Alternative Dehydrogenation Processes" (Study Number 4192 OD, 1993, 430 Ferguson Drive, Mountain View, California, 94043-5272, USA).

Eine geeignete Reaktorform ist der Festbettrohr- oder Rohrbündelreaktor. Bei diesen befindet sich der Katalysator (Dehydrierungskatalysator und, bei Arbeiten mit Sauerstoff als Co-Feed, gegebenenfalls spezieller Oxidationskatalysator) als Festbett in einem Reaktionsrohr oder in einem Bündel von Reaktionsrohren. Die Reaktionsrohre werden üblicherweise dadurch indirekt beheizt, dass in dem die Reaktionsrohre umgebenden Raum ein Gas, z.B. ein Kohlenwasserstoff wie Methan, verbrannt wird. Günstig ist es dabei, diese indirekte Form der Aufheizung lediglich auf den ersten ca. 20 bis 30% der Länge der Festbettschüttung anzuwenden und die verbleibende Schüttungslänge durch die im Rahmen der indirekten Aufheizung freigesetzte Strahlungswärme auf die erforderliche Reaktionstemperatur aufzuheizen. Übliche Reaktionsrohr-Innendurchmesser betragen etwa 10 bis 15 cm. Ein typischer Dehydrierrohrbündelreaktor umfasst ca. 300 bis 1000 Reaktionsrohre. Die Temperatur im Reaktionsrohrinneren bewegt sich üblicherweise im Bereich von 300 bis 1200°C, vorzugsweise im Bereich von 600 bis 1000°C. Der Arbeitsdruck liegt üblicherweise zwischen 0,5 und 8 bar, häufig zwischen 1 und 2 bar bei Verwendung einer geringen Wasserdampfverdünnung (analog dem BASF-Linde-Verfahren zur Propan-Dehydrierung), aber auch zwischen 3 und 8 bar bei Verwendung einer hohen Wasserdampfverdünnung (analog dem sogenannten "steam active reforming process" (STAR-Prozess) zur Dehydrierung von Propan oder Butan von Phillips Petroleum Co., siehe US 4,902,849, US 4,996,387 und US 5,389,342). Typische Katalysatorbelastungen (GHSV) liegen bei 500 bis 2000 h⁻¹. Die Katalysatorgeometrie kann beispielsweise kugelförmig oder zylindrisch (hohl oder voll) sein.

Die katalytische Ethan-Dehydrierung kann auch, wie in Chem. Eng. Sci. 1992 b, 47 (9-11) 2313 beschrieben, heterogen katalysiert im Wirbelbett durchgeführt werden, wobei Ethan nicht verdünnt wird. Zweckmäßigerweise werden dabei zwei Wirbelbetten nebeneinander betrieben, von denen sich eines in der Regel im Zustand der Regenerierung befindet. Der Arbeitsdruck beträgt typischerweise 1 bis 2 bar, die Dehydriertemperatur in der Regel 550 bis 600°C. Die für die Dehydrierung erforderliche Wärme wird dabei in das Reaktionssystem eingebracht, indem der Dehydrierkatalysator auf die Reaktionstemperatur vorerhitzt wird. Durch die Zumischung eines Sauerstoff enthaltenden Co-Feeds kann auf die Vorerhitzer verzichtet werden, und die benötigte Wärme direkt im Reaktorsystem durch Verbrennung von Wasserstoff in Gegenwart von Sauerstoff erzeugt werden. Gegebenenfalls kann zusätzlich ein Wasserstoff enthaltender Co-Feed zugemischt werden.

Die katalytische Ethan-Dehydrierung kann in einem Hordenreaktor durchgeführt werden. Dieser enthält ein oder mehrere aufeinanderfolgende Katalysatorbetten. Die Anzahl der Katalysatorbetten kann 1 bis 20, zweckmäßigerweise 1 bis 6, bevorzugt 1 bis 4 und insbesondere 1 bis 3 betragen. Die Katalysatorbetten werden vorzugsweise radial oder axial vom Reaktionsgas durchströmt. Im allgemeinen wird ein solcher Hordenreaktor mit einem Katalysatorfestbett betrieben. Im einfachsten Fall sind die Katalysatorfestbetten in einem Schachtofenreaktor axial oder in den Ringspalten von zentrisch ineinander gestellten zylindrischen Gitterrosten angeordnet. Ein Schachtofenreaktor entspricht einer Horde. Die Durchführung der Dehydrierung in einem einzelnen Schachtofenreaktor entspricht einer bevorzugten Ausführungsform. In einer weiteren bevorzugten Ausführungsform wird die Dehydrierung in einem Hordenreaktor mit 3 Katalysatorbetten durchgeführt. Bei einer Fahrweise ohne Sauerstoff als Co-Feed wird das Reaktionsgasgemisch im Hordenreaktor auf seinem Weg von einem Katalysatorbett zum nächsten Katalysatorbett einer Zwischenerhitzung unterworfen, z.B. durch Überleiten über mit heißen Gasen erhitzte Wärmeaustauscherflächen oder durch Durchleiten durch mit heißen Brenngasen beheizte Rohre.

In einer bevorzugten Ausführungsform des erfindungsgemäßen Verfahrens wird die katalytische Ethan-Dehydrierung autotherm durchgeführt. Dazu wird dem Reaktionsgasgemisch der Ethan-Dehydrierung in mindestens einer Reaktionszone zusätzlich Sauerstoff zugemischt und der in dem Reaktionsgasgemisch enthaltene Wasserstoff verbrannt, wodurch zumindest ein Teil der benötigten Dehydrierwärme in der mindestens einen Reaktionszone direkt in dem Reaktionsgasgemisch erzeugt wird. Ein Merkmal der autothermen Fahrweise gegenüber einer sogenannten oxidativen Fahrweise ist beispielsweise das Vorhandensein von Wasserstoff im Austragsgas. Bei den sogenannten oxidativen Verfahren wird kein freier Wasserstoff in wesentlichen Mengen gebildet.

Im allgemeinen wird die Menge des dem Reaktionsgasgemisch zugesetzten sauerstoffhaltigen Gas so gewählt, dass durch die Verbrennung des im Reaktionsgasgemisch vorhandenen Wasserstoffs und gegebenenfalls von im Reaktionsgasgemisch vorliegenden Kohlenwasserstoffen und/oder von in Form von Koks vorliegendem Kohlenstoff die für die Dehydrierung des Ethans benötigte Wärmemenge erzeugt wird. Im allgemeinen beträgt die insgesamt zugeführte Sauerstoffmenge, bezogen auf die Gesamtmenge des Ethans, 0,001 bis 0,5 mol/mol, bevorzugt 0,005 bis 0,2 mol/mol, besonders bevorzugt 0,05 bis 0,2 mol/mol. Sauerstoff kann entweder als reiner Sauerstoff oder als sauerstoffhaltiges Gas im Gemisch mit Inertgasen eingesetzt werden. Bevorzugtes sauerstoffhaltiges Gas ist Luft. Die Inertgase und die resultierenden Verbrennungsgase wirken im allgemeinen zusätzlich verdünnend und fördern damit die heterogen katalysierte Dehydrierung.

Die Ethan-Dehydrierung kann auch autotherm bei ca. 1000°C und einer Kontaktzeit von wenigen ms an einem Platin-Katalysator durchgeführt werden. Beispielsweise werden bei einem Ethan-Umsatz von ca. 70 % Ethen-Selektivitäten von ca. 65 % erreicht, ca. 25 % des umgesetzten Ethans zu CO und CO₂ oxidiert, ca. 5 % zu Methan und ca. 5 % zu höheren Kohlenwasserstoffen wie Propan und Propen umgesetzt. Der Einsatz eines Platin/Zinn-Katalysators an Stelle von Platin alleine bringt eine Erhöhung der Ethen-Selektivität auf ca. 70 % mit sich (Chem. Eng. Sci. 54 (1999) 765-773).

Die oxidative Ethan-Dehydrierung kann auch unter Einspeisung von Wasserstoff, vorzugsweise im Verhältnis (H₂:O₂) 2:1, z.B. in Gegenwart von Platin/Zinn/Aluminiumoxid-Katalysatoren durchgeführt werden. In diesem Fall erhöht sich die Ethen-Selektivität auf ca. 85% (Beretta, Chem. Eng. Sci. 1999, 54, 765-773; L.D. Schmidt et al. American Institute of Chemical Engineers Journal 2000, Vol. 46 (8) 1492-1495).

Der zur Wärmeerzeugung verbrannte Wasserstoff ist der bei der katalytischen Ethan-Dehydrierung gebildete Wasserstoff sowie gegebenenfalls dem Reaktionsgasgemisch zusätzlich zugesetzter Wasserstoff. Vorzugsweise sollte soviel Wasserstoff zugegen sein, dass das Molverhältnis H₂/O₂ im Reaktionsgasgemisch unmittelbar nach der Einspeisung von Sauerstoff 2 bis 10 mol/mol beträgt. Dies gilt bei mehrstufigen Reaktoren für jede Zwischeneinspeisung von Sauerstoff und gegebenenfalls Wasserstoff.

Die Wasserstoffverbrennung erfolgt katalytisch. Der eingesetzte Dehydrierungskatalysator katalysiert im allgemeinen auch die Verbrennung der Kohlenwasserstoffe und von Wasserstoff mit Sauerstoff, so dass grundsätzlich kein von diesem verschiedener spezieller Oxidationskatalysator erforderlich ist. In einer Ausführungsform wird in Gegenwart eines oder mehrerer Oxidationskatalysatoren gearbeitet, die selektiv die Verbrennung von Wasserstoff zu Sauerstoff in Gegenwart von Ethan und Ethen katalysieren. Die Verbrennung dieser Kohlenwasserstoffe mit Sauerstoff zu CO und CO₂ läuft dadurch nur in untergeordnetem Maße ab, was sich deutlich positiv auf die erzielten Selektivitäten für die Ethen-Bildung auswirkt. Vorzugsweise liegen der Dehydrierungskatalysator und der Oxidationskatalysator in verschiedenen Reaktionszonen vor.

Bei mehrstufiger Reaktionsführung kann der Oxidationskatalysator in nur einer, in mehreren oder in allen Reaktionszonen vorliegen.

Bevorzugt ist der Katalysator, der selektiv die Oxidation von Wasserstoff katalysiert, an den Stellen angeordnet, an denen höhere Sauerstoffpartialdrucke herrschen als an anderen Stellen des Reaktors, insbesondere in der Nähe der Einspeisungsstelle für das sauerstoffhaltige Gas. Die Einspeisung von sauerstoffhaltigem Gas und/oder Wasserstoff kann an einer oder mehreren Stelle des Reaktors erfolgen.

In einer Ausführungsform des erfindungsgemäßen Verfahrens erfolgt eine Zwischeneinspeisung von sauerstoffhaltigem Gas und von Wasserstoff vor jeder Horde eines Hordenreaktors. In einer weiteren Ausführungsform des erfindungsgemäßen Verfahrens erfolgt die Einspeisung von sauerstoffhaltigem Gas und von Wasserstoff vor jeder Horde außer der ersten Horde. In einer Ausführungsform ist hinter jeder Einspeisungsstelle eine Schicht aus einem speziellen Oxidationskatalysator vorhanden, gefolgt von einer Schicht aus dem Dehydrierungskatalysator. In einer weiteren Ausführungsform ist kein spezieller Oxidationskatalysator vorhanden. Die Dehydriertemperatur beträgt im allgemeinen 400 bis 1100°C, der Druck im letzten Katalysatorbett des Hordenreaktors im allgemeinen 0,2 bis 5 bar, bevorzugt 1 bis 3 bar. Die Belastung (GHSV) beträgt im allgemeinen 500 bis 2000 h⁻¹, bei Hochlastfahrweise auch bis zu 100 000 h⁻¹, bevorzugt 4000 bis 16 000 h⁻¹.

Ein bevorzugter Katalysator, der selektiv die Verbrennung von Wasserstoff katalysiert, enthält Oxide oder Phosphate, ausgewählt aus der Gruppe bestehend aus den Oxiden oder Phosphaten von Germanium, Zinn, Blei, Arsen, Antimon oder Bismut. Ein weiterer bevorzugter Katalysator, der die Verbrennung von Wasserstoff katalysiert, enthält ein Edelmetall der VIII. oder I. Nebengruppe.

Die eingesetzten Dehydrierungskatalysatoren weisen im allgemeinen einen Träger und eine Aktivmasse auf. Der Träger besteht dabei aus einem wärmebeständigen Oxid oder Mischoxid. Bevorzugt enthalten die Dehydrierungskatalysatoren ein Metalloxid, das ausgewählt ist aus der Gruppe bestehend aus Zirkondioxid, Zinkoxid, Aluminiumoxid, Siliciumdioxid, Titandioxid, Magnesiumoxid, Lanthanoxid, Ceroxid und deren Gemischen, als Träger. Bei den Gemischen kann es sich um physikalische Mischungen oder auch um chemische Mischphasen wie Magnesium- oder Zinkaluminiumoxid-Mischstrukturen handeln. Bevorzugte Träger sind Zirkondioxid und/oder Siliziumdioxid, besonders bevorzugt sind Gemische aus Zirkondioxid und Siliziumdioxid.

Die Aktivmasse der Dehydrierungskatalysatoren enthält im allgemeinen ein oder mehrere Elemente der VIII. Nebengruppe, bevorzugt Platin und/oder Palladium, besonders bevorzugt Platin. Darüber hinaus können die Dehydrierungskatalysatoren ein oder mehrere Elemente der I. und/oder II. Hauptgruppe aufweisen, bevorzugt Kalium und/oder Cäsium. Weiterhin können die Dehydrierungskatalysatoren ein oder mehrere Elemente der III. Nebengruppe einschließlich der Lanthaniden und Actiniden enthalten, bevorzugt Lanthan und/oder Cer. Schließlich können die Dehydrierungskatalysatoren ein oder mehrere Elemente der III. und/oder IV. Hauptgruppe aufweisen, bevorzugt ein oder mehrere Elemente aus der Gruppe bestehend aus Bor, Gallium, Silizium, Germanium, Zinn und Blei, besonders bevorzugt Zinn.

In einer bevorzugten Ausführungsform enthält der Dehydrierungskatalysator mindestens ein Element der VIII. Nebengruppe, mindestens ein Element der I. und/oder II. Hauptgruppe, mindestens ein Element der III. und/oder IV. Hauptgruppe und mindestens ein Element der III. Nebengruppe einschließlich der Lanthaniden und Actiniden.

Beispielsweise können erfindungsgemäß alle Dehydrierkatalysatoren eingesetzt werden, die in den WO 99/46039, US 4,788,371, EP-A 705 136, WO 99/29420, US 5,220,091, US 5,430,220, US 5,877,369, EP 0 117 146, DE-A 199 37 106, DE-A 199 37 105 und DE-A 199 37 107 offenbart werden. Besonders bevorzugte Katalysatoren für die vorstehend beschriebenen Varianten der autothermen Ethan-Dehydrierung (Stufe A) sind die Katalysatoren gemäß den Beispiel 1, 2, 3 und 4 der DE-A 199 37 107.

Die Ethan-Dehydrierung wird bevorzugt in Gegenwart von Wasserdampf durchgeführt. Der zugesetzte Wasserdampf dient als Wärmeträger und unterstützt die Vergasung von organischen Ablagerungen auf den Katalysatoren, wodurch der Verkokung der Katalysatoren entgegengewirkt und die Standzeit des Katalysators erhöht wird. Dabei werden die organischen Ablagerungen in Kohlenmonoxid und Kohlendioxid umgewandelt.

Der Dehydrierungskatalysator kann in an sich bekannter Weise regeneriert werden. So kann dem Reaktionsgasgemisch Wasserdampf zugesetzt werden oder von Zeit zu Zeit ein Sauerstoff enthaltendes Gas bei erhöhter Temperatur über die Katalysatorschüttung geleitet werden und der abgeschiedene Kohlenstoff abgebrannt werden.

Das in der Ethan-Dehydrierung eingesetzte Ethan muss nicht chemisch rein sein. Beispielsweise kann das eingesetzte Ethan bis zu 50 Vol.-% weitere Gase wie Methan, Propan, Propen, Propin, Acetylen, H₂S, SO₂, Butane, Butene sowie in Spuren organische Stickstoff- oder Schwefelverbindungen enthalten. Das eingesetzte Rohethan enthält im allgemeinen wenigstens 90 Vol.-%, vorzugsweise wenigstens 95 Vol.-% und besonders bevorzugt wenigstens 98 Vol.-% Ethan. Optional kann Ethan in einem sogenannten De-Ethanizer von höhersiedende Nebenkomponenten durch Absorption, Extraktion oder Destillation abgetrennt werden.

Bei der Ethan-Dehydrierung wird ein Gasgemisch erhalten, das neben Ethen und unumgesetztem Ethan Nebenbestandteile enthält. Übliche Nebenbestandteile sind Methan, Wasserstoff, Wasser, Stickstoff, CO und CO₂. Die Zusammensetzung des die Dehydrierstufe verlassenden Gasgemischs kann abhängig von der Fahrweise der Dehydrierung stark variieren. So wird bei Durchführung der bevorzugten autothermen Dehydrierung unter Einspeisung von Sauerstoff und zusätzlichem Wasserstoff das Produktgasgemisch einen vergleichsweise hohen Gehalt an Wasser und Kohlenstoffoxiden aufweisen. Bei Fahrweisen ohne Einspeisung von Sauerstoff wird das Produktgasgemisch der Dehydrierung einen vergleichsweise hohen Gehalt an Wasserstoff aufweisen. Beispielsweise enthält im Falle der Dehydrierung von Ethan das den Dehydrierreaktor verlassende Produktgasgemisch wenigstens die Bestandteile Ethan, Ethen und molekularen Wasserstoff. Darüber hinaus wird es in der Regel aber auch noch N₂, H₂O, Methan, CO und CO₂ enthalten. Üblicherweise wird es unter einem Druck von 0,3 bis 10 bar stehen und häufig eine Temperatur von 400 bis 1200°C, in günstigen Fällen von 500 bis 800°C, aufweisen.

Der Produktgasstrom der autothermen Ethan-Dehydrierung enthält typischerweise 10 bis 70 Vol.% Ethan, 5 bis 60 Vol.-% Ethen, 0 bis 20 Vol.-% Wasserstoff, 5 bis 50 Vol.-% Wasserdampf, 0 bis 50 Vol.-% Methan und 0 bis 20 Vol.-% Kohlenstoffoxide.

Optional kann Wasserstoff aus dem Produktgasgemisch der Ethan-Dehydrierung entfernt werden, indem er in Gegenwart eines geeigneten selektiv wirkenden Oxidationskatalysators unter Zumischung von Sauerstoff oder Luft, bevorzugt von Sauerstoff, verbrannt wird.

In den Verfahrensteilen (B1) und (B2) wird der Ethan und Ethen enthaltende Dehydrier-Produktgasstrom in mehreren Teilströmen in mehrere Chlorierungszonen eingespeist und wird Ethen zu 1,2-Dichlorethan chloriert.

Die Ethen-Chlorierung wird in dem Verfahrensteil (B1) als Oxychlorierung und in dem Verfahrensteil (B2) als Direktchlorierung durchgeführt.

Die Ethen-Chlorierung wird zum einen als Oxichlorierung durchgeführt werden. Der in die Oxichlorierungszone eingespeiste Produktgasstrom enthält neben Ethen und nicht umgesetztem Ethan vorzugsweise alle Nebenbestandteile aus der Ethan-Dehydrierung.

Durch die Verdünnung von Ethen mit Ethan und den Nebenbestandteilen im Einsatzgasstrom der Oxichlorierung wird eine höhere Selektivität der 1,2-Dichlorethan-Bildung, bezogen auf eingesetztes Ethen, erzielt und werden weniger chlorierte Nebenprodukte und Kohlenstoffoxide gebildet.

Die Oxichlorierung von Ethen zu 1,2-Dichlorethan kann grundsätzlich in allen aus dem Stand der Technik bekannten Reaktortypen und Fahrweisen durchgeführt werden.

Die Oxichlorierung von Ethen wird im allgemeinen in der Gasphase in Gegenwart eines Kupfer(II)chlorid enthaltenden Trägerkatalysators in Festbett- oder Fließbett-Fahrweise durchgeführt. Die Oxichlorierungsreaktion kann unter Zumischung von Sauerstoff oder von Luft durchgeführt werden. Vorzugsweise wird sie unter Zumischung von reinem Sauerstoff durchgeführt. Andernfalls fallen große Mengen Stickstoff an, die aus dem Verfahren ausgeschleust werden müssen und die nur schwer von mitgerissenen chlororganischen Verbindungen zu reinigen sind. Auch die Bildung von sogenannten "hot spots" im Oxichlorierungsreaktor wird durch das Arbeiten mit reinem Sauerstoff eher vermieden. Das Molverhältnis Chlorwasserstoff:Sauerstoff (HCl:O₂) beträgt im allgemeinen von 5:1 bis 3:1, vorzugsweise 4,5:1 bis 3,2:1, insbesondere 3,99:1 bis 3,5:1. Das Molverhältnis Ethylen:HCl beträgt im allgemeinen ca. 1:2. Vorzugsweise liegt Chlorwasserstoff, bezogen auf die Reaktion 2C₂H₄ + 4HCl + O₂ → 2C₂H₄Cl₂ + 2H₂O, in leicht unterstöchimetrischer Menge vor, so dass sichergestellt ist, dass Chlorwasserstoff in einem Reaktordurchgang nahezu vollständig umgesetzt wird. Das Verhältnis Ethylen:HCl:O₂ wird ferner vorzugsweise so gewählt, dass auch Ethylen in einem Reaktordurchgang möglichst weitgehend umgesetzt wird. Das Verhältnis der Kohlenwasserstoffe (Ethan, Ethen und gegebenenfalls Methan) zu Sauerstoff sollte derart sein, dass ein fettes Reaktionsgemisch oberhalb der Explosionsgrenze vorliegt. Das Molverhältnis (Ethen + Ethan):HCl:O₂ kann beispielsweise in Fließbett-Fahrweise ohne nennenswerte Anteile weiterer Kohlenwasserstoffe 1:1,19:0,31 oder 1:0,71:1,8 betragen.

Geeignete Katalysatoren enthalten in der Regel eine Kupferverbindung, vorzugsweise ein Kupferchlorid oder eine Kupferverbindung, die sich unter den Reaktionsbedingungen ganz oder teilweise in ein Kupferchlorid umwandelt, auf einem Träger. Bevorzugte Träger sind Aluminiumoxide, beispielsweise γ-Al₂O₃. Die Katalysatoren können Promotoren wie Alkalimetallchloride, insbesondere KCl, Erdalkalimetallchloride, insbesondere MgCl₂, oder Chloride der Seltenerdmetalle, insbesondere CeCl₃ enthalten. Die Oxichlorierung wird im allgemeinen bei Temperaturen von 200 bis 300°C und Drücken von 2,5 bis 7 bar durchgeführt. Der HCl-Umsatz beträgt typischerweise von 95 bis 99,9 %.

Festbettverfahren zur Oxichlorierung von Ethen zu 1,2-Dichlorethan werden beispielsweise in den DE-A 100 03 510, US 4,206,180 und GB-A 2,298,197 beschrieben. Katalysatoren zur Oxichlorierung von Ethen zu 1,2-Dichlorethan in Festbettverfahren werden beispielsweise in den EP-A 1 127 618, US 4,366,093 und EP-A 1 053 789 beschrieben.

Wirbelschichtverfahren zur Oxichlorierung von Ethen zu 1,2-Dichlorethan werden beispielsweise in den DE-A 197 53 165 A1, EP-A 1 023 939 und EP-A 0 029 143 beschrieben. Katalysatoren zur Oxichlorierung von Ethen zu 1,2-Dichlorethan in Wirbelschichtverfahren werden beispielsweise in den EP-A 0 582 165, EP-A 0 375 202 und EP-A 0 657 212 beschrieben.

Die Oxichlorierung kann auch wie in der DE-A 100 03 510 beschrieben an einer Festbett-Katalysatorschüttung, die kein separates Inertmaterial zur Verdünnung enthält, durchgeführt werden.

Der Produktgasstrom der Oxichlorierung enthält 1,2-Dichlorethan, Ethan, Wasser, im allgemeinen Spuren von Chlorwasserstoff, Ethen und Sauerstoff sowie Nebenprodukte der Oxichlorierung wie Kohlendioxid, Kohlenmonoxid sowie chlorierte Nebenprodukte wie Chloral, Vinylchlorid, 1,2-Dichlorethen, Chlorethan, 1,1-Dichlorethan, 1,1,2-Trichlorethan, Tertrachlorethan, Chloroform und Tertachlorkohlenstoff. Bezogen auf umgesetztes Ethylen werden in der Regel nicht mehr als 4%, vorzugsweise nicht mehr als 1,5% chlorierte Nebenprodukte und in der Regel nicht mehr als 5%, vorzugsweise nicht mehr als 2% Kohlenstoffoxide gebildet. Daneben kann der Produktgasstrom weitere, aus der Ethan-Dehydrierung stammende Nebenbestandteile enthalten, beispielsweise Methan, Wasserstoff, Stickstoff und zusätzliche Mengen Sauerstoff, CO und CO₂.

Aus dem Produktgasstrom der Oxichlorierung wird 1,2-Dichlorethan und ein Ethan enthaltender Gasstrom gewonnen, wobei letzterer als Kreisgas in die Ethan-Dehydrierung zurückgeführt wird. Dazu werden die kondensierbaren Anteile des Produktgasstromes, enthaltend 1,2-Dichlorethan, Wasser und Chlorwasserstoff sowie chlorierte Nebenbestandteile, auskondensiert. Üblicherweise wird der Kondensationsschritt bei einer Temperatur von 10 bis 100°C und einem Druck von 2 bis 5 bar durchgeführt. Vorzugsweise wird die Kondensation in zwei Schritten in zwei aufeinanderfolgenden Quench-Türmen ("heiß" und "kalt") und unter Zusatz von verdünnter Natronlauge zur Neutralisation von nicht umgesetztem Chlorwasserstoff im zweiten Quench-Turm durchgeführt. Es werden eine wässrige, leicht salzsaure Kondensatphase und eine organische Kondensatphase erhalten, die in einem Phasenseparator getrennt werden. Die wässrige Phase wird gegebenenfalls neutralisiert und aus dem Verfahren ausgeschleust. Die organische Phase wird destilliert, beispielsweise in einer Serie von zwei Destillationskolonnen, wobei in einer ersten Kolonne Leichtsieder über Kopf abgetrennt und in einer zweiten Kolonne reines 1,2-Dichlorethan über Kopf abgetrennt wird.

Der Ethan und die nicht kondensierbaren Nebenbestandteile enthaltende Gasstrom kann ohne Abtrennung der Nebenbestandteile als Ethan enthaltender Kreisgasstrom in die Ethan-Dehydrierung zurückgeführt werden. Der Kreisgasstrom enthält im allgemeinen noch Kohlenstoffoxide und geringe Mengen von Ethen und/oder Sauerstoff sowie Spuren chlorierter organischer Nebenprodukte der Oxichlorierung, die nicht vollständig auskondensiert wurden, beispielsweise Vinylchlorid. Der Gehalt des Kreisgasstroms an diesen chlorierten Nebenbestandteilen beträgt im allgemeinen 10 bis 10 000 ppm. Der Kreisgasstrom kann ferner Wasserstoff, Methan und/oder (bei Einsatz von Luft statt Sauerstoff) Stickstoff sowie weitere inerte Nebenbestandteile (Argon) aus der Ethan-Dehydrierung und/oder der Oxichlorierung enthalten. Um eine Anreicherung dieser unter den Verfahrensbedingungen vollständig inerten Bestandteile im Kreisgas zu vermeiden, können diese über einen Purge-Strom aus dem Verfahren ausgeschleust werden.

In einer Ausführungsform des erfindungsgemäßen Verfahrens wird also aus dem Produktgasstrom der Oxichlorierung ein Ethan und nicht kondensierbare Nebenbestandteile, ausgewählt aus der Gruppe bestehend aus Ethen, Methan, Kohlenstoffoxiden, Sauerstoff und gegebenenfalls Stickstoff und/oder Wasserstoff, enthaltender Kreisgasstrom, der noch Spuren organischer Chlorverbindungen enthalten kann, abgetrennt und dieser in die Ethan-Dehydrierung zurückgeführt.

In einer Ausführungsform des erfindungsgemäßen Verfahrens wird aus dem Kreisgasstrom Kohlendioxid durch CO₂-Gaswäsche abgetrennt. Der Kohlendioxid-Gaswäsche kann eine gesonderte Verbrennungsstufe vorgeschaltet werden, in der Kohlenmonoxid selektiv zu Kohlendioxid oxidiert wird, bevorzugt in Gegenwart von im Kreisgasstrom vorhandenem Restsauerstoff.

Bei autothermer Fahrweise der Ethan-Dehydrierung können gegebenenfalls vorhandenes Methan oder Chlorkohlenwasserstoffe über Kohlenmonoxid zu Kohlendioxid oxidiert und das Oxidationsprodukt Kohlendioxid durch die CO₂-Gaswäsche aus dem Verfahrenskreislauf ausgeschleust werden.

In einer weiteren Ausführungsform des Verfahrens kann eine Abtrennung von Nebenbestandteilen aus einem im wesentlichen Ethan enthaltenden Strom auch durch andere übliche Trennverfahren wie Destillation, Rektifikation, Membranverfahren oder Adsorption durchgeführt werden. In einer bevorzugten Ausführungsform des erfindungsgemäßen Verfahrens wird aus dem die nicht kondensierbaren Anteile enthaltenden Gasstrom in einem Absorptions-/Desorptions-Cyclus mittels eines hochsiedenden Absorptionsmittels ein Ethan enthaltender Gasstrom abgetrennt und dieser als Kreisgasstrom in die Ethan-Dehydrierungsstufe zurückgeführt. Auf diese Weise werden im wesentlichen alle nicht-kondensierbaren Nebenbestandteile (Stickstoff, Argon, Wasserstoff, Methan, Kohlenstoffoxide, Sauerstoff) aus dem Verfahrenskreislauf ausgeschleust.

Dazu wird in einer Absorptionsstufe das Ethan in einem inerten Absorptionsmittel absorbiert, wobei ein mit Ethan beladenes Absorptionsmittel und ein die übrigen Nebenbestandteile enthaltendes Abgas erhalten werden. In einer Desorptionsstufe wird Ethan aus dem Absorptionsmittel wieder freigesetzt.

In der Absorptionsstufe eingesetzte inerte Absorptionsmittel sind im allgemeinen hochsiedende unpolare Lösungsmittel, in denen das abzutrennende Alkan/Alken-Gemisch eine deutlich höhere Löslichkeit als die übrigen Bestandteile der Produktgasmischung aufweisen. Die Absorption kann durch einfaches Durchleiten der Produktgasmischung durch das Absorptionsmittel erfolgen. Sie kann aber auch in Kolonnen oder in Rotationsabsorbem erfolgen. Dabei kann im Gleichstrom, Gegenstrom oder Kreuzstrom gearbeitet werden. Geeignete Absorptionskolonnen sind z.B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht- und Dünnschichtabsorber sowie Rotationskolonnen, Tellerwäscher, Kreuzschleierwäscher und Rotationswäscher in Betracht.

Geeignete Absorptionsmittel sind vergleichsweise unpolare organische Lösungsmittel, beispielsweise aliphatische C₈- bis C₁₈-Alkene, oder aromatische Kohlenwasserstoffe wie die Mittelölfraktionen aus der Paraffindestillation, oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁-C₈-Alkanolen, wie Benzoesäure-n-butylester, Benzoesäuremethylester, Benzoesäureethylester, Phthalsäuredimethylester, Phthalsäurediethylester, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%. Geeignete Absorptionsmittel sind ferner Octane, Nonane, Decane, Undecane, Dodecane, Tridecane, Tetradecane, Pentadecane, Hexadecane, Heptadecane und Octadecane oder aus Raffinerieströmen gewonnene Fraktionen, die als Hauptkomponenten die genannten linearen Alkane enthalten.

Zur Desorption wird das beladene Absorptionsmittel erhitzt und/oder auf einen niedrigeren Druck entspannt. Alternativ dazu kann die Desorption auch durch Strippung oder in einer Kombination von Entspannung, Erhitzen und Strippung in einem oder mehreren Verfahrensschritten erfolgen. Das in der Desorptionsstufe regenerierte Absorptionsmittel wird in die Absorptionsstufe zurückgeführt.

Aus dem Absorptions/Desorptions-Zyklus wird ein Kreisgasstrom erhalten, der im wesentlichen aus Ethan besteht, wenn der Absorptions/Desorptions-Zyklus nach der Chlorierung und vor der (erneuten) Dehydrierung erfolgt, und der im wesentlichen aus Ethan und Ethen besteht, wenn der Absorptions/Desorptions-Zyklus nach der Dehydrierung und vor der Chlorierung erfolgt. Im allgemeinen enthält auch dieser Kreisgasstrom noch in Spuren chlorierte organische Nebenprodukte wie Vinylchlorid, im allgemeinen in Mengen von 10 bis 10 000 ppm.

Überraschender Weise bewirken die organischen Chlorverbindungen in dem in die Ethan-Dehydrierung zurückgeführten Kreisgas eine Erhöhung der Aktivität und der Standzeit des Dehydrierungskatalysators.

In einer weiteren Ausführungsform des erfindungsgemäßen Verfahrens wird also aus dem Produktgasstrom der Oxichlorierung ein Ethan und nicht kondensierbare Nebenbestandteile, ausgewählt aus der Gruppe bestehend aus Ethen, Methan, Kohlenstoffoxiden, Sauerstoff, Stickstoff, Wasserstoff und Spuren organischer Chlorverbindungen enthaltender Gasstrom abgetrennt, aus diesem Gasstrom in einem Absorptions-/Desorptions-Cyclus mittels eines hochsiedenden Absorptionsmittels ein im wesentlichen aus Ethan bestehender Kreisgasstrom, der noch Spuren organischer Chlorverbindungen enthalten kann, abgetrennt und dieser in die Ethan-Dehydrierungsstufe zurückgeführt.

Die Chlorierung wird zum anderen als Direktchlorierung durchgeführt.

Die aus dem Einsatzgasstrom der Ethan-Dehydrierung stammenden und die bei der Ethan-Dehydrierung gebildeten Nebenbestandteile können vor der Direktchlorierung aus dem Einsatzgasstrom der Direktchlorierung abgetrennt werden.

Zur Abtrennung des im Produktgasgemisch der Ethan-Dehydrierung enthaltenen Wasserstoffs kann das Produktgasgemisch, gegebenenfalls nach erfolgter Kühlung, beispielsweise in einem indirekten Wärmetauscher, über eine in der Regel als Rohr ausgebildete Membran geleitet werden, die lediglich für molekularen Wasserstoff durchlässig ist. Der so abgetrennte molekulare Wasserstoff kann bei Bedarf zumindest teilweise in der Dehydrierung eingesetzt oder aber einer sonstigen Verwertung zugeführt werden, beispielsweise zur Erzeugung elektrischer Energie in Brennstoffzellen eingesetzt werden.

Alternativ kann die Wasserstoffabtrennung auch durch partielle Kondensation, Adsorption und/oder Rektifikation, vorzugsweise unter Druck, vorgenommen werden.

Wasserstoff kann auch, wie oben beschrieben, an einem geeigneten Katalysator selektiv verbrannt werden.

Wasserstoff kann auch gemeinsam mit weiteren Nebenbestandteilen aus dem Produktgasgemisch der Ethan-Dehydrierung abgetrennt werden.

Die Abtrennung von Wasserstoff kann erforderlich sein, um die Ausbildung explosiver Wasserstoff/Sauerstoff- oder Wasserstoff/Chlor-Gasgemische in der Direktchlorierung zu vermeiden.

In einer Ausführungsform des erfindungsgemäßen Verfahrens wird zunächst Wasser abgetrennt. Die Wasserabtrennung vor der Direktchlorierung kann vorteilhaft sein, um eine Beeinträchtigung des bei der Direktchlorierung eingesetzten Katalysators zu vermeiden und/oder um bei der Aufarbeitung der Produkte der Direktchlorierung die Ausbildung einer wässrigen Phase zu vermeiden. Das Abtrennen von Wasser kann beispielsweise durch Auskondensieren durch Abkühlen und/oder Verdichten des Produktgasstroms der Dehydrierung erfolgen und kann in einer oder mehreren Abkühlungs- und/oder ' Verdichtungsstufen durchgeführt werden. Die Wasserabtrennung wird üblicherweise durchgeführt, wenn die Alkan-Dehydrierung autotherm durchgeführt oder unter Einspeisung von Wasserdampf isotherm (analog dem Linde- oder STAR-Prozess zur Dehydrierung von Propan) durchgeführt wird und folglich der Produktgasstrom einen hohen Wasseranteil aufweist.

Nach der Wasserabtrennung können Ethan und Ethen in einem Absorptions-/Desorptions-Zyklus mittels eines hochsiedenden Absorptionsmittels von den übrigen Nebenbestandteilen abgetrennt werden. Dazu werden in einer Absorptionsstufe Ethan und Ethen in einem inerten Absorptionsmittel absorbiert, wobei ein mit Ethan und Ethen beladenes Absorptionsmittel und ein die Nebenbestandteile enthaltendes Abgas erhalten werden, und in einer Desorptionsstufe Ethan und Ethen aus dem Absorptionsmittel freigesetzt.

Die Abtrennung von im wesentlichen allen von Ethan und Ethen verschiedenen Nebenbestandteilen aus dem Produktgasgemisch erfolgt vorzugsweise dadurch, dass das auf Temperaturen von 10 bis 70°C abgekühlte Produktgasgemisch bei einem Druck von im allgemeinen 0,1 bis 50 bar, bevorzugt 2 bis 20 bar und einer Temperatur von im allgemeinen 0 bis 100°C, bevorzugt 5 bis 80°C mit einem vorzugsweise hochsiedenden unpolaren organischen Lösungsmittel, in welchem Ethan und Ethen absorbiert werden, in Kontakt gebracht wird. Durch nachfolgende Desorption, beispielsweise durch Strippung, werden Ethan und Ethen wieder freigesetzt und der Direktchlorierung zugeführt.

Der Absorptions/Desorptions-Zyklus wird im übrigen wie oben beschrieben durchgeführt, wobei als Absorptionsmittel vorzugsweise die oben genannten hochsiedenden Absorptionsmittel eingesetzt werden. Vorzugsweise werden beide Absorptions/Desorptions-Zyklen, also die Abtrennung der Nebenbestandteile aus dem Produktgasstrom der Oxichlorierung einerseits und der Nebenbestandteile aus dem Produktgasstrom der Ethan-Dehydrierung andererseits, in einem gemeinsamen Lösungsmittelkreislauf durchgerührt.

Die Direktchlorierung von Ethen mit Chlor zu 1,2-Dichlorethan kann grundsätzlich in allen bekannten Reaktortypen und nach allen bekannten Fahrweisen durchgeführt werden. Ein geeignetes Verfahren zur Herstellung von 1,2-Dichlorethan durch direkte Chlorierung wird beispielsweise in der US 4,873,384 beschrieben.

Die Direktchlorierung kann in flüssiger Phase durchgeführt werden, vorzugsweise wird sie so durchgeführt. Die Direktchlorierung kann als Hochtemperatur-Direktchlorierung in Gegenwart von FeCl₃ als Homogenkatalysator in 1,2-Dichlorethan als Reaktionsmedium bei einer Temperatur von 120 bis 130°C und einem Druck von ca. 3 bar durchgeführt werden. Dazu werden Chlor und das Ethan/Ethen-Gemisch, das gegebenenfalls Nebenbestandteile enthält, in das flüssige Reaktionsmedium eingeleitet. Das Molverhältnis Ethen : Chlor ist üblicherweise nahe 1, wobei Ethen in leichtem Überschuss vorliegt, und beträgt beispielsweise 1,01:1 bis 1,1:1. Die Direktchlorierung kann auch als Niedrigtemperatur-Direktchlorierung in Gegenwart eines Komplexkatalysators in 1,2-Dichlorethan durchgeführt werden. Durch die entstehende Reaktionswärme verdampft ein Teil des Reaktionsmediums.

Die Direktchlorierung kann auch als Gasphasen-Direktchlorierung bei ca. 250°C und 1,4 bar an einem Aluminiumoxid-Katalysator in Fließbettfahrweise durchgeführt werden.

Der Ethen-Umsatz der Direktchlorierung liegt im allgemeinen bei nahe 99 %, der Chlor-Umsatz bei nahe 100 %. Die Selektivität der 1,2-Dichlorethan-Bildung beträgt in der Regel ca. 99 %. Die Direktchlorierung wird vorzugsweise in Gegenwart geringer Mengen Sauerstoff durchgeführt, um die Bildung chlorierter Nebenprodukte zurückzudrängen.

Der Produktgasstrom der Direktchlorierung enthält 1,2-Dichlorethan, Ethan und gegebenenfalls weitere Nebenbestandteile. Aus diesem werden 1,2-Dichlorethan und ein Ethan enthaltender Kreisgasstrom gewonnen, der in die Ethan-Dehydrierung zurückgeführt wird.

Zur Gewinnung von 1,2-Dichlorethan werden die kondensierbaren Anteile des Produktgasstroms auskondensiert. Dabei können eine wässrige und eine organische Phase erhalten werden, die in einem Phasenseparator getrennt werden. Üblicherweise erfolgt, falls der Produktgasstrom der Ethan-Dehydrierung nennenswerte Wasseranteile enthält, eine Wasserabtrennung vor der Direktchlorierung, so dass im Kondensationsschritt nach der Direktchlorierung keine wässrige Phase anfällt. Der Kondensationsschritt wird im allgemeinen bei einer Temperatur von 80 bis 140°C, vorzugsweise Raumtemperatur, und einem Druck von 3 bis 6 bar durchgeführt. Aus der organische Phase wird, beispielsweise in einer Serie von zwei Destillationskolonnen, reines 1,2-Dichlorethan gewonnen.

Die nicht kondensierbaren Anteile des Produktgasstromes enthalten Ethan, unumgesetztes Ethen, gegebenenfalls Sauerstoff und/oder Wasserstoff, in Spuren nicht vollständig auskondensierte chlorierte Nebenprodukte der Direktchlorierung und, falls eine Abtrennung vor der Direktchlorierung nicht erfolgt ist, noch nicht-kondensierbare Nebenbestandteile aus der Ethan-Dehydrierung. Die Spuren der chlorierten Nebenprodukte liegen in der Größenordnung von 10 bis 10 000 ppm.

In einer Ausführungsform der Erfindung wird vor der Direktchlorierung keine Abtrennung von nicht-kondensierbaren Nebenbestandteilen der Ethan-Dehydrierung durchgeführt und werden die nicht kondensierbaren Anteile des Produktgasstroms der Direktchlorierung als Ethan enthaltender Kreisgasstrom in die Ethan-Dehydrierung zurückgeführt. Dabei kann, wie oben beschrieben, Kohlendioxid durch Gaswäsche, gegebenenfalls in Verbindung mit einer vorgeschalteten Kohlenmonoxid-Verbrennung, aus dem Kreisgasstrom abgetrennt werden.

Durch die Verdünnung von Ethen mit nicht umgesetztem Ethan und gegebenenfalls nicht kondensierbaren Nebenbestandteilen im Einsatzgasstrom der Direktchlorierung werden höhere Raum/Zeit-Ausbeuten und höhere Selektivitäten der 1,2-Dichlorethan-Bildung erzielt, insbesondere bei Durchführung der Flüssigphasen-Direktchlorierung in einer Blasensäule.

In einer Variante des erfindungsgemäßen Verfahrens wird aus dem zweiten Teilstrom des Dehydrier-Produktgasstroms zunächst Wasser abgetrennt und werden anschließend Ethan und Ethen in einem Absorptions-/Desorptions-Zyklus mittels eines hochsiedenden Absorptionsmittels von den übrigen Nebenbestandteilen abgetrennt, und werden Ethan und Ethen in die Direktchlorierungszone eingespeist.

In einer weiteren Variante des erfindungsgemäßen Verfahrens werden aus dem Produktgasstrom der Oxichlorierung und aus dem Produktgasstrom der Direktchlorierung jeweils ein Ethan und nicht kondensierbare Nebenbestandteile, ausgewählt aus der Gruppe bestehend aus Ethen, Sauerstoff, Methan, Kohlenstoffoxiden, Stickstoff und Wasserstoff, enthaltender Kreisgasstrom, der noch Spuren von Chlorkohlenwasserstoffen enthalten kann, abgetrennt und diese werden in die Ethan-Dehydrierung zurückgeführt. Vorzugsweise werden diese Kreisgasströme vereinigt und einer Kohlendioxid-Gaswäsche, gegebenenfalls mit vorgeschalteter Kohlenmonoxid-Verbrennung, unterzogen.

In einer weiteren Variante des erfindungsgemäßen Verfahrens werden aus dem Produktgasstrom der Oxichlorierung und aus dem Produktgasstrom der Direktchlorierung jeweils ein Ethan und nicht kondensierbare Nebenbestandteile, ausgewählt aus der Gruppe bestehend aus Ethen, Sauerstoff, Methan, Kohlenstoffoxiden, Stickstoff und Wasserstoff sowie Spuren organischer Chlorverbindungen, enthaltender Gasstrom abgetrennt. Aus diesem Gasstrom wird ein im wesentlichen aus Ethan bestehender Kreisgasstrom, der noch Spuren organischer Chlorverbindungen enthalten kann, vorzugsweise in einem Absorptions-/Desorptions-Zyklus mittels eines hochsiedenden Absorptionsmittels abgetrennt und in die Ethan-Dehydrierung zurückgeführt. Vorzugsweise werden die Gasströme vor der Ethan-Abtrennung vereinigt.

In den genannten Varianten erfolgt die Aufarbeitung der nicht-kondensierbaren organischen Anteile aus der Oxichlorierung und der Direktchlorierung bevorzugt gemeinsam.

Spuren von organischen Chlorverbindungen können in einem Absorptionsschritt mit geeigneten, dem Fachmann bekannten Absorptionsmitteln aus dem Kreisgasstrom abgetrennt werden.

Das bei der Oxichlorierung und der Direktchlorierung von Ethen erhaltene 1,2-Dichlorethan wird einer Pyrolyse zu Vinylchlorid und Chlorwasserstoff unterworfen. Der bei der Pyrolyse gebildete Chlorwasserstoff wird in die Oxichlorierung zurückgeführt.

Die Pyrolyse wird in der Gasphase, beispielsweise bei 500°C und Drücken von 6 bis 21 bar, durchgeführt.

Es wird ein Produktgasstrom enthaltend Vinylchlorid, Chlorwasserstoff, 1,2-Dichlorethan und gegebenenfalls Nebenbestandteile erhalten. Aus diesem werden Chlorwasserstoff und Vinylchlorid abgetrennt. Nicht umgesetztes 1,2-Dichlorethan kann in die Pyrolyse zurückgeführt werden, wobei eine gemeinsame Aufarbeitung von abgetrenntem Roh-1,2-Dichlorethan mit aus der Oxichlorierung und der Direktchlorierung stammendem Roh-1,2-Dichlorethan zur Gewinnung von reinem 1,2-Dichlorethan erfolgen kann.

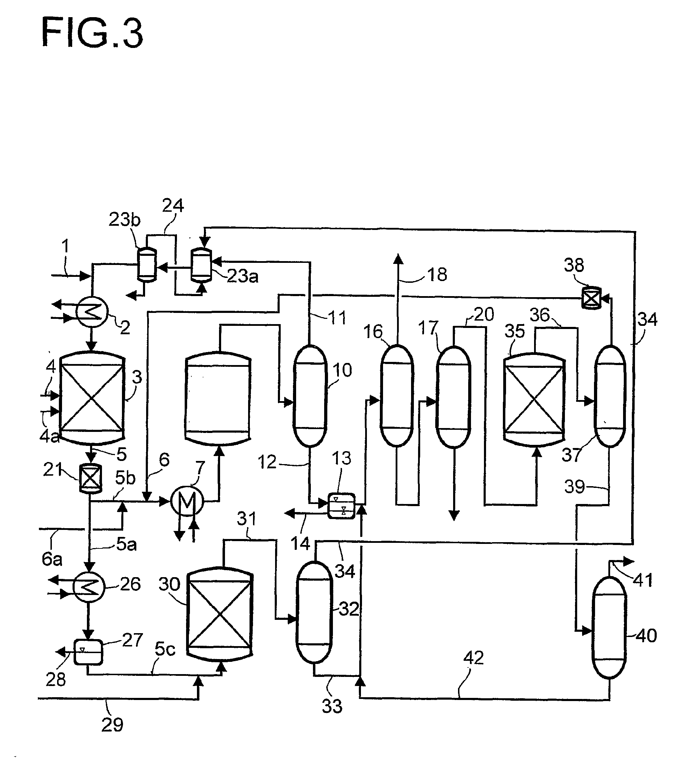

Das erfindungsgemäße Verfahren wird anhand der Figur 3 näher erläutert.

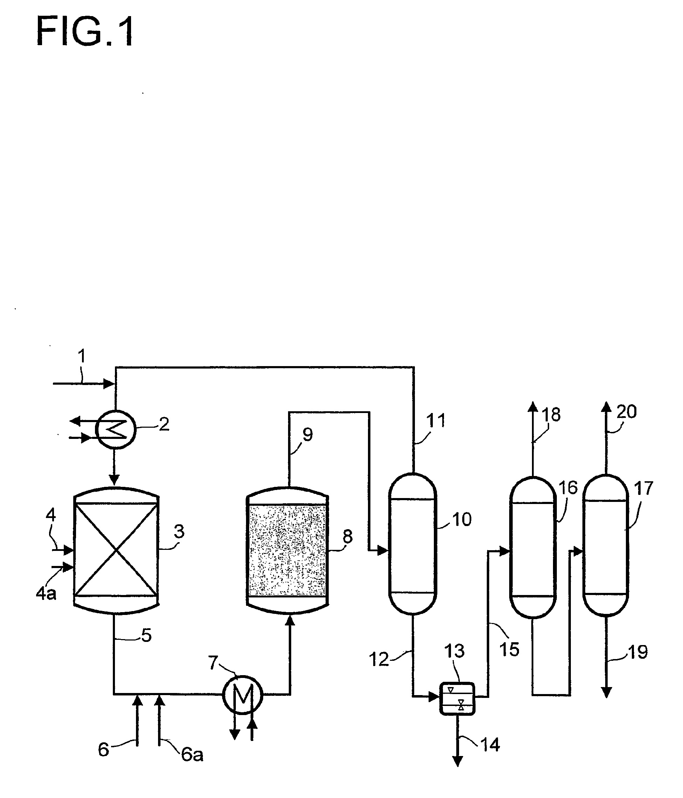

Figur 1 zeigt ein Fließbild eines nicht erfindungsgemäßen Verfahrens. Der Ethan enthaltende Einsatzgasstrom 1 wird mit dem Ethan enthaltenden Kreisgasstrom 11 gemischt, in einem Wärmetauscher 2 auf die erforderliche Reaktoreintrittstemperatur vorgeheizt und in den Dehydrierreaktor 3 eingespeist. In den Dehydrierreaktor 3 wird optional Sauerstoff oder Luft als Co-Feed 4 und/oder Wasserstoff als Co-Feed 4b eingespeist. Dem Ethen enthaltenden Produktgasstrom 5 der Dehydrierung werden Chlorwasserstoff 6 und Sauerstoff 6a zugemischt. Das Gasgemisch wird in einem Wärmetauscher 7 auf die erforderliche Eintrittstemperatur gebracht und in den Oxichlorierungsreaktor 8 eingespeist. Der Produktgasstrom 9 der Oxichlorierung wird einem Kondensator 10 zugeleitet und in einen nicht kondensierbare Bestandteile enthaltenden Gasstrom 11 und kondensierbare Bestandteilen enthaltenden Strom 12 aufgetrennt. Der die nicht kondensierbaren Bestandteile enthaltende Gasstrom 11 wird als Kreisgasstrom in die Ethan-Dehydrierung zurückgeführt. Der die kondensierbaren Bestandteile enthaltende Strom 12 wird in einem Phasenseparator 13 in eine wässrige Phase 14 und eine organische Phase 15 getrennt. Die wässrige Phase wird aus dem Verfahren ausgeschleust. Die organischen Phase 15 wird in einer Serie von zwei Destillationskolonnen 16 und 17 aufgetrennt, wobei am Kopf der ersten Kolonne eine Leichtsiederfraktion 18, am Sumpf der zweiten Kolonne eine Schwersiederfraktion 19 und am Kopf der zweiten Kolonne reines 1,2-Dichlorethan 20 erhalten wird.

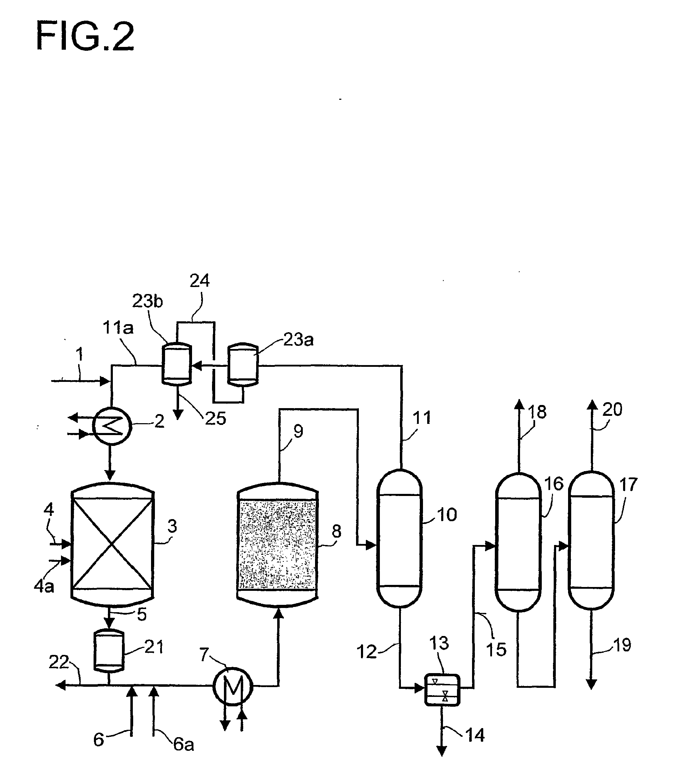

Figur 2 zeigt ein Fließbild eines weiteren nicht erfindungsgemäßen Verfahrens. Im Unterschied zu der in Figur 1 dargestellten Ausführungsform wird hier in einem dem Dehydrierreaktor nachgeschalteten Wasserstoff-Verbrenner 21 Wasserstoff selektiv zu Wasser oxidiert. Optional kann ein Teilgasstrom 22 abgezogen und in einem anderen Verfahren, beispielsweise der Ethylenoxid-Herstellung oder der Ethylbenzol-Herstellung, als Einsatzgasstrom eingesetzt werden. Aus dem die nicht kondensierbaren Bestandteile 11 enthaltenden Gasstrom wird in einer Absorptions/Desorptions-Vorrichtung 23 a,b mittels eines im Kreis geführten hochsiedenden Lösungsmittels 24 ein Ethan und Ethen enthaltender Kreisgasstrom 11 a von den übrigen nicht kondensierbaren Bestandteilen 25 abgetrennt. Die nicht kondensierbaren Bestandteile werden aus dem Verfahren ausgeschleust.
Figur 3 zeigt ein Fließbild einer Ausführungsform des erfindungsgemäßen Verfahrens. Der Produktgasstrom der Ethan-Dehydrierung 5 wird in zwei Teilströme 5a und 5b aufgeteilt. Der Teilstrom 5a wird in einem Wärmetauscher 26 abgekühlt und einem Kondensator 27 zugeleitet, in dem ein Wasserstrom 28 auskondensiert wird. Der im wesentlichen wasserfreie Gasstrom 5c wird mit einem Chlorgasstrom 29 gemischt und in den Direktchlorierungsreaktor 30 eingespeist. Der Produktgasstrom 31 der Direktchlorierung wird in einem Kondensator 32 in einen nicht kondensierbare Bestandteile enthaltenden Gasstrom 34 und einen kondensierbare Bestandteile enthaltenden Strom 33 aufgetrennt. Der die kondensierbaren Bestandteile enthaltende Strom 33 wird gemeinsam mit dem die kondensierbaren organischen Bestandteile der Oxichlorierungsreaktion enthaltenden Strom 15 in den Destillationskolonnen 16 und 17 gemeinsam aufgearbeitet, wobei reines 1,2-Dichlorethan 20 erhalten wird. Der die nicht kondensierbaren Bestandteilen enthaltende Gasstrom 34 wird der Absorptions/Desorptions-Vorrichtung 23 a,b zugeleitet, in der aus den Gasströmen 11 und 34 Ethan und Ethen von den übrigen nicht kondensierbaren Bestandteilen abgetrennt werden. Der am Kopf der zweiten Destillationskolonne 17 abgezogene 1,2-Dichlorethanstrom wird in den Pyrolysereaktor 35 eingespeist. Aus dem Produktgasstrom 36 der 1,2-Dichlorethan-Pyrolyse wird in einer Destillationskolonne 37 ein Chlorwasserstoffstrom über Kopf abgetrennt. In diesem gegebenenfalls enthaltenes Acetylen wird in einem Hydrierreaktor 38 hydriert und der von Acetylen befreite Chlorwasserstoffstrom als Gasstrom 6 in die Oxichlorierung zurückgeführt. Der am Sumpf der Destillationskolonne 37 erhaltene, die flüssigen Produkte der 1,2-Dichlorethan-Pyrolyse enthaltende Strom 39 wird in eine Destillationskolonne 40 eingeleitet. Am Kopf der Destillationskolonne 40 wird Roh-Vinylchlorid 41 gewonnen, das weiter aufgereinigt werden kann. Am Sumpf dieser Kolonne wird ein 1,2-Dichlorethan enthaltender Strom 42 erhalten, welcher mit den die kondensierbaren Bestandteile der Direktchlorierung enthaltenden Strom 33 und dem die kondensierbaren organischen Bestandteile der Oxichlorierung enthaltenden Strom 15 in den Destillationskolonnen 16 und 17 gemeinsam aufgearbeitetwird.

## Patentansprüche

1. Verfahren zur Herstellung von 1,2-Dichlorethan mit den Schritten
(A) Einspeisung eines Ethan enthaltenden Einsatzgasstroms in eine Dehydrierzone, Dehydrierung von Ethan zu Ethen unter Erhalt eines Produktgasstromes enthaltend Ethan, Ethen und Nebenbestandteile,
(B1) Einspeisung eines ersten Teilstroms des Ethan und Ethen enthaltenden Dehydrier-Produktgasstroms, gegebenenfalls nach Abtrennung von Nebenbestandteilen, in eine Oxichlorierungszone, Oxichlorierung von Ethen zu 1,2-Dichlorethan unter Erhalt eines 1,2-Dichlorethan, Ethan und gegebenenfalls weitere Nebenbestandteile enthaltenden Produktgasstroms, Gewinnung von 1,2-Dichlorethan und eines Ethan enthaltenden Kreisgasstroms und Rückführung des Ethan enthaltenden Kreisgasstroms in die Ethan-Dehydrierung,
(B2) Einspeisung eines zweiten Teilstroms des Ethan und Ethen enthaltenden Dehydrier-Produktgasstroms, gegebenenfalls nach Abtrennung von Nebenbestandteilen, in eine Direktchlorierungszone, direkte Chlorierung von Ethen zu 1,2-Dichlorethan unter Erhalt eines 1,2-Dichlorethan, Ethan und gegebenenfalls weitere Nebenbestandteile enthaltenden Produktgasstroms, Gewinnung von 1,2-Dichlorethan und eines Ethan enthaltenden Kreisgasstroms und Rückführung des Ethan enthaltenden Kreisgasstroms in die Ethan-Dehydrierung,
(C) Einspeisung von 1,2-Dichlorethan aus der Oxichlorierung und der Direktchlorierung in eine Pyrolysezone, Pyrolyse von 1,2-Dichlorethan zu Vinylchlorid unter Erhalt eines Vinylchlorid, Chlorwasserstoff, 1,2-Dichlorethan und gegebenenfalls Nebenbestandteile enthaltenden Produktgasstromes, Gewinnung von Vinylchlorid und Rückführung von Chlorwasserstoff in die Oxichlorierung.

2. Verfahren nach Anspruch 1, **dadurch gekennzeichnet, dass** die Dehydrierung (Schritt A) durch thermische Spaltung (Cracken), oxidative Dehydrierung oder nicht-oxidative katalytische Dehydrierung erfolgt.

3. Verfahren nach Anspruch 2, **dadurch gekennzeichnet, dass** die Dehydrierung durch autotherme katalytische Dehydrierung erfolgt.

4. Verfahren nach einem der Ansprüche 1 bis 3, **dadurch gekennzeichnet, dass** aus dem zweiten Teilstrom des Dehydrier-Produktgasstroms zunächst Wasser abgetrennt und anschließend Ethan und Ethen in einem Absorptions-/Desorptions-Cyclus mittels eines hochsiedenden Absorptionsmittels von den übrigen Nebenbestandteilen abgetrennt werden, und Ethan und Ethen in die Direktchlorierungszone eingespeist werden.

5. Verfahren nach einem der Ansprüche 1 bis 4, **dadurch gekennzeichnet, dass** aus dem Produktgasstrom der Oxichlorierung ein Ethan und nicht kondensierbare Nebenbestandteile, ausgewählt aus der Gruppe bestehend aus Ethen, Methan, Kohlenstoffoxiden, Stickstoff und Wasserstoff, enthaltender Kreisgasstrom abgetrennt und dieser in die Ethan-Dehydrierung zurückgeführt wird.

6. Verfahren nach einem der Ansprüche 1 bis 5, **dadurch gekennzeichnet, dass** aus dem Produktgasstrom der Oxichlorierung ein Ethan und nicht kondensierbare Nebenbestandteile, ausgewählt aus der Gruppe bestehend aus Ethen, Methan, Kohlenstoffoxiden, Stickstoff und Wasserstoff, enthaltender Gasstrom abgetrennt wird, aus diesem Gasstrom in einem Absorptions-/Desorptions-Cyclus mittels eines hochsiedenden Absorptionsmittels ein im wesentlichen aus Ethan bestehender Kreisgasstrom abgetrennt und dieser in die Ethan-Dehydrierungsstufe zurückgeführt wird.

7. Verfahren nach Ansprüche 1 bis 3, **dadurch gekennzeichnet, dass** aus dem Produktgasstrom der Oxichlorierung und aus dem Produktgasstrom der Direktchlorierung jeweils ein Ethan und nicht kondensierbare Nebenbestandteile, ausgewählt aus der Gruppe bestehend aus Ethen, Methan, Kohlenstoffoxiden, Stickstoff und Wasserstoff, enthaltender Kreisgasstrom abgetrennt wird und dieser in die Ethan-Dehydrierung zurückgeführt wird.

8. Verfahren nach einem der Ansprüche 5 oder 7, **dadurch gekennzeichnet, dass** Kohlendioxid aus dem Kreisgasstrom durch Kohlendioxid-Gaswäsche entfernt wird.

9. Verfahren nach Anspruch 8, **dadurch gekennzeichnet, dass** Kohlenmonoxid in einer vorgeschalteten Verbrennungsstufe zu Kohlendioxid oxidiert wird.

10. Verfahren nach einem der Ansprüche 1 bis 3, **dadurch gekennzeichnet, dass** aus dem Produktgasstrom der Oxichlorierung und aus dem Produktgasstrom der Direktchlorierung jeweils ein Ethan und nicht kondensierbare Nebenbestandteile, ausgewählt aus der Gruppe bestehend aus Ethen, Methan, Kohlenstoffoxiden, Stickstoff und Wasserstoff, enthaltender Gasstrom abgetrennt wird, aus diesem Gasstrom in einem Absorptions-/Desorptions-Cyclus mittels eines hochsiedenden Absorptionsmittels ein im wesentlichen aus Ethan bestehender Gasstrom abgetrennt wird und dieser als Kreisgasstrom in die Ethan-Dehydrierung zurückgeführt wird.

## Claims

1. The process for preparing 1,2-dichloroethane which comprises
(A) feeding an ethane-comprising feed gas stream into a dehydrogenation zone and dehydrogenating ethane to ethene to give a product gas stream comprising ethane, ethene and secondary constituents,
B1) feeding a first substream of the ethane- and ethene-comprising dehydrogenation product gas stream, if appropriately after having separated off secondary constituents, into an oxychlorination zone, oxychlorinating ethene to 1,2-dichloroethane to give a product gas stream comprising 1,2-dichloroethane, ethane and if appropriate further secondary constituents, isolating 1,2-dichloroethane and an ethane-comprising circulating gas stream and recirculating the ethane-comprising circulating gas stream to the ethane dehydrogenation,
(B2) feeding a second substream of the ethane- and ethene-comprising dehydrogenation product gas stream, if appropriate after having separated off secondary constituents, into a direct chlorination zone, directly chlorinating ethene to 1,2-dichloroethane to give a product gas stream comprising 1,2-dichloroethane, ethane and if appropriate further secondary constituents, isolating 1,2-dichloroethane and an ethane-comprising circulating gas stream and recirculating the ethane-comprising circulating gas stream to the ethane dehydrogenation,
(C) feeding 1,2-dichloroethane from the oxychlorination and from the direct chlorination into a pyrolysis zone, pyrolyzing 1,2-dichloroethane to vinyl chloride to give a product gas stream comprising vinyl chloride, hydrogen chloride, 1,2-dichloroethane and is appropraite secondary constituents, isolating vinyl chloride and recirculating hydrogen chloride to the oxychlorination.

2. The process according to claim 1, wherein the dehydrogenation (step A) is carried out by thermal cracking, oxidative dehydrogenation or nonoxidative catalytic dehydrogenation.

3. The process according to claim 2, wherein the dehydrogenation is carried out by autothermal catalytic dehydrogenation.

4. The process according to any of claims 1 to 3, wherein water is firstly separated off from the second substream of the dehydrogenation product gas stream and ethane and ethene are subsequently separated off from the other secondary constituents by means of a high-boiling absorption medium in an absorption/desorption cycle, and ethane and ethene are fed into the direct chlorination zone.

5. The process according to any of claims 1 to 4, wherein a circulating gas stream comprising ethane and incondensable secondary constituents selected from the group consisting of ethene, methane, carbon oxides, nitrogen and hydrogen is separated off from the product gas stream from the oxychlorination and is recirculated to the ethane dehydrogenation.

6. The process according to any of claims 1 to 5, wherein a gas stream comprising ethane and incondensable secondary constituents selected from the group consisting of ethene, methane, carbon oxides, nitrogen and hydrogen is separated off from the product gas stream from the oxychlorination, a circulating gas stream consisting essentially of ethane is separated off from this gas stream by means of a high-boiling absorption medium in an absorption/desorption cycle and this circulating gas stream is recirculated to the ethane dehydrogenation stage.

7. The process according to claims 1 to 3, wherein a circulating gas stream comprising ethane and incondensable secondary constituents selected from the group consisting of ethene, methane, carbon oxides, nitrogen and hydrogen is in each case separated off from the product gas stream from the oxychlorination and from the product gas stream from the direct chlorination and is recirculated to the ethane dehydrogenation.

8. The process according to claim 5 or 7, wherein carbon dioxide is removed from the circulating gas stream by means of a carbon dioxide gas scrub.

9. The process according to claim 8, wherein carbon monoxide is oxidized to carbon dioxide in an upstream combustion stage.

10. The process according to any of claims 1 to 3, wherein a gas stream comprising ethane and incondensable secondary constituents selected from the group consisting of ethene, methane, carbon oxides, nitrogen and hydrogen is in each case separated off from the product gas stream from the oxychlorination and from the product gas stream from the direct chlorination, a gas stream consisting essentially of ethane is separated off from this gas stream by means of a high-boiling absorption medium in an absorption/desorption cycle and this gas stream consisting essentially of ethane is recirculated as circulating gas stream to the ethane dehydrogenation.

## Revendications

1. Procédé de préparation de 1,2-dichloroéthane avec les étapes suivantes :
(A) introduction d'un flux de gaz contenant de l'éthane dans une zone de déshydrogénation, déshydrogénation d'éthane en éthène avec obtention d'un flux de gaz produit contenant de l'éthane, de l'éthène et des constituants secondaires;
(B1) introduction d'un premier flux partiel du flux de gaz produit de la déshydrogénation contenant l'éthane et de l'éthène, le cas échéant après séparation des constituants secondaires, dans une zone d'oxychloration, oxychloration d'éthène en 1,2-dichloroéthane avec obtention d'un flux de gaz produit contenant du 1,2-dichloroéthane, de l'éthane et le cas échéant encore d'autres constituants secondaires, obtention de 1,2-dichloroéthane et d'un flux de gaz cyclique contenant de l'éthane et recyclage du flux de gaz cyclique contenant de l'éthane dans la déshydrogénation de l'éthane;
(B2) introduction d'un deuxième flux partiel du flux de la gaz produit de la déshydrogénation contenant de l'éthane et de l'éthène, le cas échéant après séparation des constituants secondaires, dans une zone de chloration directe, chloration directe d'éthène en 1,2-dichloroéthane avec obtention d'un flux de gaz produit contenant du 1,2-dichloroéthane, de l'éthane et le cas échéant d'autres constituants secondaires, obtention de 1,2-dichloroéthane et d'un flux de gaz cyclique contenant de l'éthane et recyclage du flux de gaz cyclique contenant de l'éthane dans la déshydrogénation de l'éthane;
(C) introduction de 1,2-dichloroéthane de l'oxychloration et de la chloration directe dans une zone de pyrolyse, pyrolyse du 1,2-dichloroéthane en chlorure de vinyle avec obtention d'un flux de gaz produit contenant du chlorure de vinyle, du chlorure d'hydrogène, du 1,2-dichloroéthane et le cas échéant d'autres constituants secondaires, obtention de chlorure de vinyle et recyclage du chlorure d'hydrogène dans l'oxychloration.

2. Procédé selon la revendication 1, **caractérisé en ce que** la déshydrogénation (étape A) s'effectue par séparation thermique (crackage), déshydrogénation oxydative ou déshydrogénation catalytique non oxydative.

3. Procédé selon la revendication 2, **caractérisé en ce que** la déshydrogénation s'effectue par déshydrogénation catalytique autothermique.

4. Procédé selon l'une quelconque des revendications 1 à 3, **caractérisé en ce que** du deuxième flux partiel du flux de gaz produit de la déshydrogénation, de l'eau d'abord est séparée des constituants secondaires restants, puis de l'éthane et de l'éthène sont séparés dans un cycle d'absorption/désorption au moyen d'un agent d'absorption à haut point d'ébullition, et de l'éthane et de l'éthène sont introduits dans la zone de chloration directe.

5. Procédé selon l'une quelconque des revendications 1 à 4, **caractérisé en ce que** du flux de gaz produit de l'oxychloration, un flux de gaz cyclique contenant de l'éthane et des constituants secondaires condensables, sélectionnés parmi le groupe composé d'éthène, de méthane, d'oxydes de carbone, d'azote et d'hydrogène, est séparé, et celui-ci est recyclé dans la déshydrogénation d'éthane.

6. Procédé selon l'une quelconque des revendications 1 à 5, **caractérisé en ce que** du flux de gaz produit de l'oxychloration est séparé un flux de gaz contenant un éthane et des constituants secondaires non condensables, sélectionnés parmi le groupe composé d'éthène, de méthane, d'oxydes de carbone, d'azote et d'hydrogène, un flux de gaz cyclique, essentiellement constitué d'éthane, est séparé de ce flux de gaz dans un cycle d'absorption/désorption au moyen d'un agent d'absorption à haut point d'ébullition, lequel flux de gaz cyclique est recyclé dans l'étape de déshydrogénation d'éthane.

7. Procédé selon les revendications 1 à 3, **caractérisé en ce que** du flux de gaz produit de l'oxychloration et du flux de gaz produit de la chloration directe, un flux de gaz cyclique contenant chaque fois de l'éthane et des constituants secondaires non condensables sélectionnés parmi le groupe composé d'éthène, de méthane, d'oxydes de carbone, d'azote et d'hydrogène, est séparé et celui-ci est recyclé dans la déshydrogénation d'éthane.

8. Procédé selon l'une des revendications 5 ou 7, **caractérisé en ce que** du dioxyde de carbone du flux de gaz cyclique est éliminé par lavage de gaz dioxyde de carbone.

9. Procédé selon la revendication 8, **caractérisé en ce que** du monoxyde de carbone est oxydé en dioxyde de carbone dans une étape de combustion agencée en aval.

10. Procédé selon l'une des revendications 1 à 3, **caractérisé en ce que** du flux de gaz produit de l'oxychloration et du flux de gaz produit de la chloration directe, est séparé chaque fois un flux de gaz contenant de l'éthane et des constituants secondaires non condensables sélectionnés parmi le groupe composé d'éthène, de méthane, d'oxydes de carbone, d'azote et d'hydrogène, de ce flux de gaz est séparé, dans un cycle d'absorption/désorption au moyen d'un agent d'absorption à haut point d'ébullition, un flux de gaz essentiellement composé d'éthane, qui est recyclé dans la déshydrogénation d'éthane.
